# EUROPEAN PATENT SPECIFICATION

(11) **EP 2 286 819 B1**
(45) Date of publication and mention of the grant of the patent: **12.06.2019**
(21) Application number: 09741698.6
(22) Date of filing: 30.04.2009
(51) Int. Cl.: A61K 31/7048, A61K 9/08, A61K 9/14, A61K 9/20, A61K 9/48, A61P 9/10

(54) **PHARMACEUTICAL COMPOSITION FOR TREATING CARDIO-CEREBRO VASCULAR DISEASES AND PREPARATIVE METHOD AND KIT THEREOF**
PHARMAZEUTISCHE ZUSAMMENSETZUNG ZUR BEHANDLUNG VON KARDIOZEREBROVASKULÄREN ERKRANKUNGEN UND HERSTELLUNGSVERFAHREN UND KIT DAFÜR
COMPOSITION PHARMACEUTIQUE DESTINÉE À TRAITER DES MALADIES CARDIOVASCULAIRES ET CÉRÉBROVASCULAIRES, PROCÉDÉ DE PRÉPARATION ET KIT CORRESPONDANTS

(30) Priority: 09.05.2008 CN 200810096184
(43) Date of publication of application: 23.02.2011
(73) Proprietor: KPC Pharmaceuticals, Inc., Kunming, Yunnan 650106 (CN)
(72) Inventor: HUANG, Zhaochang, Yunnan 650106 (CN); YANG, Zhaoxiang, Yunnan 650106 (CN); PU, Junxue, Yunnan 650106 (CN); LIU, Yidan, Yunnan 650106 (CN); ZHENG, Shuangqing, Yunnan 650106 (CN); GONG, Yunqi, Yunnan 650106 (CN)
(74) Representative: Detken, Andreas
(86) International application number: PCT/CN2009/071579
(87) International publication number: WO 2009/135423

(56) References cited:
- WO-A2-2004/091645
- CN-A- 1 857 285
- CN-A- 101 040 863
- CN-A- 101 062 088
- CN-A- 101 129 395
- CN-A- 101 357 136
- CN-C- 100 384 429

## Description

### FIELD OF INVENTION

The invention relates to a pharmaceutical composition for use in the treatment or prevention of cardio-cerebro vascular diseases. In particular, the present invention relates to a pharmaceutical composition comprising ginsenoside Rb₁ and ginsenoside Rg₁ as the active ingredients. The invention also relates to a method for preparing the pharmaceutical composition and a kit containing the same.

### BACKGROUND OF THE INVENTION

Cardio-cerebro vascular diseases are the primary diseases threatening human health. According to the global statistics on death of various diseases made by World Health Organization (WHO) in 1996, cardio-cerebro vascular diseases rank as the diseases leading to the highest mortality and disability, with a patient population of at least about 450 millions. A total of 14 millions people died of coronary heart disease, cerebral stroke and other heart disease, occupying 28.8% of total deaths. The mortality showed an increasing trend year by year. According to incomplete statistics, people died of cardio-cerebro vascular diseases accounted for 34% of deaths in 2000, accounting for about half of all deaths due to diseases.

The situation in China is generally similar to the statistics made by the WHO. The deaths caused by cardiovascular disease accounted for 39.4% of the total deaths in 1999, and cardiovascular disease is the top disease and the main reason leading to death. There are more than 6 million cerebral stroke patients in China, in which 75% of patients lost the ability to work in varying degrees, and 4% of patients are severely disabled. There are about 1.5 million new stroke patients and 750,000 new patients with coronary heart disease every year. Furthermore, there are more patients suffering from hypertension or hyperlipidemia disease, the number of which has already surpassed 100 million now. The family burden, medical and economic burdens resulted therefrom have become a social problem that can not be underestimated. Cardio-cerebro vascular diseases have become one of the worldwide major public health problems, thus, development of new drugs for treatment of cardio-cerebro vascular diseases has become extremely urgent.

Ginsenoside Rb₁, one of the principal ingredients of total saponin of *Panax notoginseng,* has the following pharmacological effects: (1) anti-aging effect, mainly including the improvement of sexual function and adrenal function, anti-stress and anti-lipid peroxidation. (2) protecting against myocardial ischemia-reperfusion injury. Ginsenoside Rb₁ has the effect of calcium channel blockade, and has significant protective effect on acute myocardial ischemia in dogs. The apoptosis of myocardial cells was significantly reduced via sublingual intravenous injection of 20mg/kg ginsenoside Rb₁ before reperfusion in rats, indicating that ginsenoside Rb₁ could inhibit apoptosis of myocardial cells during ischemia-reperfusion and reduce myocardial ischemia reperfusion injury. (3) protecting against lung ischemia-reperfusion injury. Ginsenoside Rb₁ could reduce lung ischemia-reperfusion injury caused by ischemia and hypoxia through regulating the tension of airway and vascular smooth muscle via the NO pathway, inhibiting capillary leakage and neutrophil adhesion. (4) protecting against cerebral ischemia injury. Ginsenoside Rb1 has protective effect on ischemic injury in exo vivo rat hippocampal slices. It could reduce the release of excitatory neurotransmitters such as glutamate induced by hypoxia from synaptic vesicle and decrease the extracellular glutamate level, thus reduce the excitotoxicity on neurons and protect nerve cells. This suggests that the mechanism of protecting against cerebral ischemia injury of ginsenoside Rb₁ has connection with its anti-excitotoxicity of glutamate. (5) Promoting regeneration of peripheral nerve cells. Ginsenoside Rb₁ could promote Schwann cells' proliferation. (6) improving learning and memory in mice. Ginsenoside Rb₁ could increase the acetylcholine content in brain and the number of M cholinergic receptor and the like. Ginsenoside Rb₁ and ginsenoside Rg₁ can both facilitate the process of learning and memory, enhance cholinergic system function and improve neural plasticity, thus improve learning and memory.

Ginsenoside Rg₁ is one of the principal ingredients of total saponins of *Panax notoginseng.* The effect of ginsenoside Rg₁ on cerebral ischemia reperfusion injury and adhesion of endothelial cells showed that ginsenoside Rg₁ could improve the cerebral ischemia-reperfusion injury by inhibiting leukocyte infiltration and adhesion molecule expression in rats. The effect of ginsenoside Rg₁ on cell apoptosis during the cerebral ischemia-reperfusion injury in rat showed that ginsenoside Rg₁ has a significant protective effect on apoptosis induced by cerebral ischemia reperfusion injury, and its mechanism may be connected with ginsenoside Rg₁ affecting the expression of Caspase-3 and Bc1-2.

The effects of ginsenoside Rg₁ on vascular endothelial growth factor (VEGF) and hypoxia-induced factor 1α (HIF-1α) after acute myocardial infarction showed that severe ischemia may stimulate the myocardium to produce large amounts of VEGF and HIF-1α, which have protective effect on ischemic myocardium. Ginsenoside Rg₁ can stimulate the angiogenesis in myocardial infarct area and the establishment of collateral circulation by increasing their expression. The effects of ginsenoside Rg₁ on brain mitochondrial function during cerebral ischemia-reperfusion injury in rat showed that ginsenoside Rg₁ has significant protective effects on cerebral ischemia-reperfusion injury and one of the possible mechanisms is the protection of mitochondrial function and anti-oxygen free radical effect. In addition, ginsenoside Rg₁ has the pharmacological effects such as immune promotion, anti-aging and intellectual promotion.

Junxian Wei etc. demonstrated that ginsenoside Rb₁ and ginsenoside Rg₁ have a similar effect with total saponins of *Panax notoginseng* in the aspect of experimental myocardial ischemia, vascular smooth muscle and the like in "Panax notoginseng-modern scientific research and application". Ginsenoside Rg₁ has significant effects on anti-platelet aggregation, promoting blood circulation such as thrombolysis, and ginsenoside Rb₁ has a good effect on reperfusion injury in thrombolytic therapy, and both are the active ingredients for the vasodilation of vascular smooth muscle.

Total saponins of *Panax notoginseng* has effects of activating blood circulation and eliminating stasis, and activating the collaterals, so it is used for the treatment of apoplexy and hemiplegia, blood stasis and sequelae of cerebrovascular disease, chest distress and cardiodynia, occlusion of central retinal vein of patients who have blood stasis syndrome in the clinic. Since the chemical components of Total saponins of Panax notoginseng is not clear, the mechanism by which they act is indefinite, thus, the rate of adverse reactions is increasing year by year. With the increasing amount used and the expanding usage, safety has become an important issue.

WO 2004/091645 A2 discloses pharmaceutical compositions for use in the treatment of cardiovascular conditions comprising rosmarinic acid, borneol and ginsenosides Rg₁ and Rb₁.

CN 101 129 395 A discloses a preparation containing a mixture of active principles of Chinese herbs for treating cardiovascular and cerebrovascular diseases, more specifically a preparation containing Rb₁ and/or Rg₁, as well as ophiopogon root saponin C (DT-13) and schizandrol.

CN 100 384 429 C discloses a composition comprising panax notoginsenosidum (Panax notoginseng saponins) that comprise at least Rg₁, Rb₁ and arasaponin R1.

CN 101 062 088 A discloses a composition comprising salvia miltiorrhizae extract (comprising tanshinone IIA, tanshinols, and salvianolic acid), a panax notoginseng root extract (comprising Rg₁, Rb₁ and R1) and a natural levo-borneol (l-Borneol).

CN 101 357 136 A discloses a composition comprising at least Rb₁, Rg₁, Rd₁, Re₁, etc.

### SUMMARY OF THE INVENTION

The present invention is defined in the appended claims and is limited thereto.

Surprisingly, the inventors found that the pharmaceutical composition containing ginsenoside Rb₁ and ginsenoside Rg₁ as the active ingredients not only has significant effects on anti-platelet aggregation and promoting blood circulation such as thrombolysis, but also can solve the most important outstanding problems of safety and controllability of total saponins of *Panax notoginseng* injections for decades and greatly improve the safety of use.

Therefore, the present invention provides a pharmaceutical composition consisting of ginsenoside Rg₁ and ginsenoside Rb₁ as active ingredients and a pharmaceutically acceptable carrier, the pharmaceutical composition being for use in the treatment or prevention of cardio-cerebro vascular diseases, wherein the weight ratio of ginsenoside Rg₁ to ginsenoside Rb₁ is 1:10-10:1, and and the total amount of ginsenoside Rg₁ and ginsenoside Rb₁ is 85% to 99.99%, based on the total weight of the pharmaceutical composition.

In another aspect, the present invention provides a method for preparing the pharmaceutical composition according to the present invention, comprising the step of combining ginsenoside Rg₁ with ginsenoside Rb₁ in a weight ratio of 1:10-10:1, wherein the purity of ginsenoside Rg₁ used is 85% to 99.99%, and the purity of ginsenoside Rb₁ used is 85% to 99.99%.

In a further aspect, the present invention provides a kit for use in the improvement, treatment or prevention of cardio-cerebro vascular diseases, wherein the kit comprises the pharmaceutical composition according to the present invention.

The pharmaceutical composition according to the present invention has the advantages such as reliable efficacy and low rate of adverse reactions, especially, its has a considerable efficacy compared with other compositions comprising ginsenoside Rg₁ and ginsenoside Rb₁ (for example, an injection of total saponins of *Panax notoginseng*), its composition is clear and fixed, and its quality is controllable, thus, the incidence of adverse reactions is significantly reduced and the safety of use is greatly improved.

### DETAILED DESCRIPTION OF THE INVENTION

One aspect of the present disclosure is to provide a pharmaceutical composition comprising ginsenoside Rg₁ and ginsenoside Rb₁ as the active ingredients for improving, treating or preventing cardio-cerebro vascular diseases, wherein the weight ratio of ginsenoside Rg₁ to ginsenoside Rb₁ is 1:10 to 10:1.

Ginsenoside Rg₁ (molecular formula: C₄₂H₇₂O₁₄, molecule weight: 801.01) has the structure as shown in formula (I):

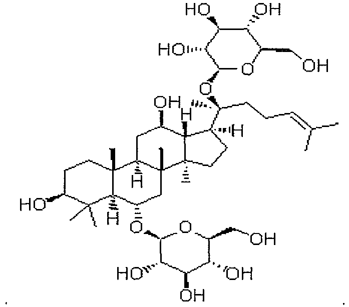

Ginsenoside Rb₁ (molecular formula: C₅₄H₉₂O₂₃, molecule weight: 1109.29) has the structure as shown in formula (II):

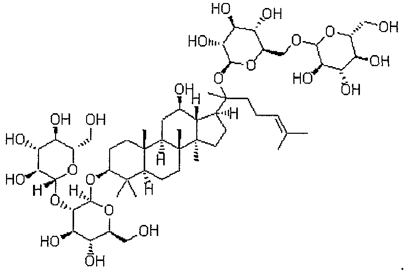

Ginsenoside Rg₁ and Rb₁ used in the present invention can be obtained according to the methods known in the art, for example, can be purified from crude medicine such as *Panax notoginseng, Panax ginseng*, American ginseng and semi-synthetic ginsenoside, can be synthesized by the methods known in the art, and also can be purchased from the market. The purities of ginsenoside Rg₁ and Rb₁ used in the present invention are usually 85% to 99.99%, preferably 90% to 99.99%, more preferably 95% to 99.99%.

The present inventors found that, when the content of ginsenoside Rb₁ contained in the pharmaceutical composition is higher than that of ginsenoside Rg₁, the resulting pharmaceutical composition has particularly notable effects of replenishing qi and quite the heart, activating blood circulation and eliminating stasis; when the content of ginsenoside Rg₁ contained in the pharmaceutical composition is higher than that of ginsenoside Rb₁, the resulting pharmaceutical composition has particularly notable effects of activating blood circulation and eliminating stasis, replenishing qi and blood circulation. When the weight ratio of ginsenoside Rg₁ to ginsenoside Rb₁ in the pharmaceutical composition is in the range from 1:10 to 10:1, the best effects of activating blood circulation and eliminating stasis, activating the collaterals can be achieved.

In some preferred embodiments of the present invention, the weight ratio of ginsenoside Rg₁ to ginsenoside Rb₁ is 3:5 to 5:1, preferably 1:1.5 to 1.5:1, more preferably about 1:1.

In some embodiments of the present invention, the pharmaceutical composition of the invention can comprise pharmaceutically acceptable carriers, and thus can be formulated into preparations of a variety of dosage forms, and can be administered to mammals by the various forms suitable for the chosen route of administration such as oral or parenteral, intravenous, intramuscular, topical or subcutaneous routes. The preparations can be the in the forms such as injection, powder for injection, enteric-coated tablets, pills, powder, granules, mixtures, syrup, capsules, dripping pills and the like.

In the present invention, the pharmaceutically acceptable carriers mean conventional drug carriers in the pharmaceutical field, such as excipients, fillers, binders, disintegrating agents, lubricants, antioxidants, coating agents, coloring agents, fragrances and surfactants.

The pharmaceutical composition of the present invention can be formulated into conventional preparations of various dosage forms in accordance with conventional preparation methods. The appropriate carriers can be chosen according to different dosage forms of pharmaceutical compositions. The preparation of the appropriate dosage forms can be achieved by the existing conventional technology for the preparation of formulations, and the formulations can also be coated if necessary.

For example, the pharmaceutical compositions of the present invention can be administered systematically such as orally with a pharmaceutically acceptable carrier, such as inert diluent. They can be encapsulated in hard shell or soft shell gelatin capsules, or can be pressed into tablets, or can be directly added to the diet of patients. For oral administration, the active compounds can be combined with one or more carriers and administered in the forms such as tablets, buccal tablets, lozenges, capsules, elixir, suspension, syrup, wafer and the like.

The preparations including tablets, lozenges, pills and capsules can also contain the following components: binders, such as tragacanth gum, arabic gum, corn starch or gelatin; excipients, such as dicalcium phosphate; disintegrating agents, such as corn starch, potato starch, alginate and the like; lubricants, such as magnesium stearate; and sweeteners, such as sucrose, fructose, lactose or aspartame; or flavoring agent, such as mint, wintergreen oil; or other perfumes can be added. When the unit dosage form is a capsule, in addition to the above-mentioned types of materials, it can also contain liquid carriers such as vegetable oil or polyethylene glycol. A variety of other substances can exist as a coating or to change the physical form of the solid unit dosage forms. For example, the preparations including tablets, pills or capsules can be coated with gelatin, wax, shellac or sugar. Syrup or elixir can contain active compounds, sucrose or fructose as a sweetener, methylparaben and propylparaben as a preservative, dyes and flavoring agent such as cherry or orange flavor. It should be noted that, any of the substances used in the preparation of any unit dosage form should be pharmaceutical acceptable and essentially non-toxic in terms of the amount used. In addition, the active compounds can be added to the sustained-release preparations and devices.

The active compounds can also be administered via infusion, intravenous injection or intraperitoneal injection. The solution of the active compounds or salts thereof can be prepared in water optionally mixed with non-toxic surfactants. Furthermore, the dispersion can also be prepared in glycerol, liquid polyethylene glycol, triacetin and mixtures thereof and in oil. In ordinary conditions of storage and use, these preparations contain preservatives to prevent microbial growth.

Dosage forms suitable for injection or infusion may include sterile aqueous solution or dispersions or sterile powder containing the active ingredient, which are suitable for temporary preparation of sterile injectable or infusion solution or dispersions, optionally encapsulated in the liposomes. In all cases, the final dosage form should be sterile, flowable and stable in the conditions of preparation and storage. Liquid carrier or vehicle can be a solvent or liquid dispersion medium containing for example water, ethanol, polyols (such as glycerol, propylene glycol, liquid polyethylene glycol, etc.), vegetable oil, non-toxic glyceride and an appropriate mixture thereof. For example, adequate flowability can be maintained by formation of liposomes, maintaining the required particle size in the case of dispersion or using surfactants. Various antibacterial agents and antifungal agents (for example, parabens, chloro-butanol, phenols, sorbic acid, thimerosal, etc.) to prevent microbial growth. In many cases, an isotonic agent such as sugar, buffer or sodium chloride is preferably comprised. An absorption-delaying agent such as aluminum monostearate or gelatin may be added to injectable compositions in order to prolong the absorption of the composition.

A sterile injectable solution can be prepared through mixing the required amount of active compounds with various other ingredients listed above in an appropriate solvent, followed by filtration sterilization, as required. In the case of preparing sterile powders for sterile injectable solution, vacuum drying and freeze-drying techniques are the preferred methods. A powder containing the active ingredients and any additional desired component present in the above sterile filtered solution is obtained.

The total amount of ginsenoside Rg₁ and Rb₁ as the active ingredients is 85% to 99.99%, preferably 87% to 98%, more preferably 90% to 95%, based on the total weight of the pharmaceutical composition.

In the second aspect of the present invention, a method for preparing the pharmaceutical composition of the present invention is provided. The method comprises the step of combining ginsenoside Rg₁ with ginsenoside Rb₁ in a weight ratio of 1:10 to 10:1.

Unless specifically mentioned, there is no special limitation on the specific process steps and process conditions in the preparation of the pharmaceutical compositions according to the present invention, and those skilled in the art can determine them based on the common knowledge in the art and/or reference is made to any conventional method known the art for preparing pharmaceutical compositions, as long as they can produce the pharmaceutical composition within the scope of the present invention.

In some preferred embodiments of the present invention, the method comprises the step of combining ginsenoside Rg₁ with ginsenoside Rb₁ in a weight ratio of 3:5 to 5:1, preferably 1:1.5 to 1.5:1, more preferably 1:1.

The method comprises the step of combining ginsenoside Rg₁ and ginsenoside Rb₁ as active ingredients with other components of the pharmaceutical composition, wherein the total weight of ginsenoside Rg₁ and ginsenoside Rb₁ is 85% to 99.99%, preferably 87% to 98%, more preferably 90% to 95%, based on the total weight of the pharmaceutical composition. The other components of the pharmaceutical compositions include pharmaceutically acceptable carriers, or essentially consisted of pharmaceutically acceptable carriers.

The pharmaceutical composition of the present invention has the efficacies of activating blood circulation and eliminating stasis, activating the collaterals etc., and can be used for the improvement, treatment or prevention of the diseases such as obstruction of collaterals by blood stasis, hemiplegia after apoplexy, sequelae of cerebrovascular disease, obstrution of Qi in the chest and precordial pain and central retinal vein occlusion.

The pharmaceutical composition of the present invention may be used according to the conventional methods of various types of formulations, and the dosage may be changed according to the route of administration, the age and body weight of patients, the type and severity of the diseases to be treated, which can be easily determined by physicians based on the his knowledge. For example, the pharmaceutical compositions or preparations of the present invention can be administered by the following methods in the following effective amount. For enteric-coated tablets, pills, powder, granules, mixtures, syrup, capsules and dripping pills, the oral dosage is 60-120mg (for example, based on the weight ratio of 1:1, the amount of ginsenoside Rg₁ and ginsenoside Rb₁ is 30-60mg respectively) once, 3 times a day for 4 weeks as a course of treatment. The injection and powder for injection can be used for intravenous drip or intramuscular injection. For intravenous drip, the dosage is 60-120 mg, once daily, and slow drip is performed after being diluted in 250-500ml of 5% or 10% glucose solution for injection. For intramuscular injection, the dosage is 60-120mg, once a day, fifteen days as a course of treatment. The second course of treatment can be preceded 1 to 3 days after withdrawal.

In the third aspect, the present invention is to provide a kit for use in the improvement, treatment or prevention of cardio-cerebro vascular diseases, wherein the kit comprises the pharmaceutical composition according to the present invention.

The pharmaceutical composition of the present invention can be present in the kit in any dosage form convenient to apply. Optionally, the kit of the invention can also comprise a drug delivery device and/or instructions. The drug delivery device is preferably a disposable delivery device, which can be for example cartridges, ampoules, syringes and/or syringe cartridges, etc., and it can also comprise other elements, such as needles, swabs and so on. For example, in the case that the pharmaceutical composition of the invention is an injection, the pharmaceutical composition of the present invention can be placed in the drug delivery device in a pre-determined amount to facilitate intramuscular injection or subcutaneous injection, and subcutaneous injection is preferred. The instructions usually involve the route how to administer the pharmaceutical composition and/or the diseases that the pharmaceutical composition can be treated.

It should be noted that, in the context of the present invention, unless otherwise indicated, all percentages refer to weight percentage. In addition, the numerical range mentioned herein contains end points.

The following preparation examples and test examples can illustrate the present invention in more detail. The following test examples are intended to verify the beneficial effects of the present invention. Specifically, the comparative studies on the primary efficacy, toxicity and hemolysis were performed to illustrate that the pharmaceutical composition of the present invention has the exact efficacies of improving, treating or preventing cardio-cerebro vascular diseases, and it has the advantages of definite active ingredients, controllable quality and obvious reduced occurrence rate of adverse reactions. The invention solves the unsolved significant problem of safety and controllability of total saponins of *Panax notoginseng* injections for decades.

Source of raw materials and specifications are as follows: *Panax notoginseng* (rhizome) was obtained from Kunming Pharmaceutical Group and the batch number was 20050812; Silica gel (200-300 mesh) was purchased from Qingdao Ocean Chemical Factory. D101 macroporous resin was purchased from Shanghai Mosu Company. Alumina was purchased from Shanghai Nahui Company.

### Preparation Example 1: preparation of the powder for injection of the composition comprising ginsenoside Rg₁ and ginsenoside Rb₁ with a purity of 99.99%

*Panax notoginseng* was crushed into 1kg of coarse powder. 5000ml of 70% ethanol was added. The mixture was extracted by refluxing for 3 times, 2 hours once, then the extract solution was collected and filtered. Ethanol was recovered from the filtrate until the smell of ethanol disappeared. Water was added thereto to obtain a solution, in which 1ml of the solution containing 0.5g crude drug. The solution was loaded on a macroporous resin column (D class resin, the volume of the resin was 4000ml, and the ratio of the height to the diameter of the resin bed was about 10), and eluted by gradient elution with water and 20%, 40%, 60% ethanol solution respectively, then fractions of 40% ethanol (A, containing ginsenoside Rg₁) and 60% ethanol (B, containing ginsenoside Rb₁) were collected and evaporated to dryness. Fraction A was dissolved in appropriate amount of methanol, then 3 times weight of silica was added. The mixture was stirred, dried and sieved over 80 mesh, then loaded on a silica gel column (the weight ratio of the sample to silica is 1:50), eluted with a solution of chloroform and methanol (8:1). The eluent was collected and TLC identification was performed. The fractions containing ginsenoside Rg₁ as a single spot were combined and evaporated to dryness, to obtain 25g of ginsenoside Rg₁, with a purity of 99.5% detected by HPLC (if the purity was below standard, the product was reloaded on silica gel column under the condition as loading of fraction A on silica gel column until the requirement was achieved). Fraction B was dissolved in appropriate amount of methanol, then 3 times weight of silica was added. The mixture was stirred, dried and sieved over 80 mesh, then loaded on a silica gel column (the weight ratio of the sample to silica is 1:50), eluted with the solution of chloroform and methanol (5:1). The eluent was collected and TLC identification was performed. The fractions containing ginsenoside Rb₁ as a single spot were combined and evaporated to dryness, to obtain 25g of ginsenoside Rb₁, with a purity of 99.6% detected by HPLC (if the purity was below standard, the product was reloaded on silica gel column under the condition as loading of fraction A on silica gel column until the requirement was achieved). The obtained ginsenoside Rg₁ and ginsenoside Rb₁ were combined in a weight ratio of 4:3. The resulting mixture was dissolved in appropriate amount of water for injection, filtrated, filled and freeze-dried, then was prepared into a powder for injection.

### Preparation Example 2: preparation of the injection of the composition comprising ginsenoside Rg₁ and ginsenoside Rb₁ with a purity of 85.0%

*Panax notoginseng* was crushed into 1kg of coarse powder. 5000ml of 50% ethanol was added. The mixture was extracted by refluxing 3 times, 2 hours once, then the extract was collected and filtered. Ethanol was collected from the filtrate until the smell of ethanol disappeared. Water was added thereto to obtain a solution, in which 1ml of the solution contained 0.5g crude drug. The solution was loaded on the macroporous resin column (D class resin, the volume of the resin was 4000ml, and the ratio of the height to the diameter of the resin bed was about 10), and eluted by gradient elution with water and 20%, 40%, 60% ethanol solution respectively, then fractions of 40% ethanol (A, containing ginsenoside Rg₁) and 60% ethanol(B, containing ginsenoside Rb₁) were collected and evaporated to dryness, and dissolved by adding appropriate amount of 95% ethanol, then loaded on a neutral alumina column (the weight radio of the sample to the alumina is 1:50). Fraction A was eluted by gradient elution with 95% and 85% ethanol. The eluent was collected and TLC identification was performed. The fractions containing ginsenoside Rg₁ as a single spot were combined and evaporated to dryness, to obtain 30g of ginsenoside Rg₁, with a purity of 85.5% detected by HPLC. Fraction B was eluted by gradient elution with 95%, 75% and 65% ethanol The eluent was collected and TLC identification was performed. The fractions containing ginsenoside Rb₁ as a single spot were combined and evaporated to dryness, to obtain 26g of ginsenoside Rb₁, with a purity of 85.6% detected by HPLC. The obtained ginsenoside Rg₁ and ginsenoside Rb₁ were combined in a weight ratio of 3:4. The resulting mixture was dissolved in appropriate amount of water for injection, ultra-filtrated, and filled, then prepared into injections.

### Preparation Example 3: preparation of the powder for injection of the composition comprising ginsenoside Rg₁ and ginsenoside Rb₁ with a purity of 90.0%

*Panax notoginseng* was crushed into 1kg of coarse powder. 5000ml of 40% ethanol was added. The mixture was extracted by refluxing for 3 times, 2 hours once, then the extract was collected and filtered. The filtration was concentrated to a solution in which 1 ml containing 1g of crude drug. 3 times volume of silica was added. The mixture was stirred, dried and sieved over 80 mesh, then loaded on a silica gel column (the weight ratio of the sample to the silica is 1:30), eluted with a solution of chloroform and methanol (8:1). The eluent was collected and TLC identification was performed. The fractions containing ginsenoside Rg₁ (fraction ginsenoside Rg₁, referred to as fraction A for short) were combined. The mixture was eluted with a solution of chloroform and methanol (8:1). The eluent was collected and TLC identification was performed. The fractions containing ginsenoside Rb₁ (fraction ginsenoside Rb₁, referred to as fraction B for short) were combined and evaporated to dryness. Fraction A was dissolved in appropriate amount of methanol, then 3 times weight of silica was added. The mixture was stirred, dried and sieved over 80 mesh, then loaded on a silica gel column (the weight ratio of the sample to the silica is 1:50), eluted with a solution of chloroform and methanol (8:1). The eluent was collected and TLC identification was performed. The fractions containing ginsenoside Rg₁ as a single spot were combined and evaporated to dryness, to obtain 28g of ginsenoside Rg₁, with a purity of 91.5% detected by HPLC. Fraction B was dissolved in appropriate amount of methanol, then 3 times the weight of silica was added. The mixture was stirred, dried and sieved over 80 mesh, then loaded on a silica gel column (the weight ratio of the sample to the silica is 1:50), eluted with a solution of chloroform and methanol (5:1). The eluent was collected and TLC identification was performed. The fractions containing ginsenoside Rb₁ as a single spot were combined and evaporated to dryness, to obtain 25g of ginsenoside Rb₁, with a purity of 92.3% detected by HPLC. The obtained ginsenoside Rg₁ and ginsenoside Rb₁ were combined in a weight ratio of 1:1. The resulting mixture was dissolved in appropriate amount of water for injection, untra-filtrated, filled and freeze-dried, then prepared into powder for injection.

### Preparation Example 4: preparation of tablets of the pharmaceutical composition of the present invention

Ginsenoside Rg₁ with a purity of 99.5% prepared in the above preparation example 1 and ginsenoside Rb₁ with a purity of 85.6% prepared in the above preparation example 2 were combined in a weight ratio of 3:5. The resulting mixture was prepared into tablets according to conventional preparation methods.

### Preparation Example 5: preparation of capsules of the pharmaceutical composition of the present invention

Ginsenoside Rg₁ with a purity of 85.5% prepared in the above preparation example 2 and ginsenoside Rb₁ with a purity of 99.6% prepared in the above preparation example 1 were combined in a weight ratio of 5:1. The resulting mixture was prepared into capsules according to conventional preparation methods.

### Preparation Example 6: preparation of dripping pills of the pharmaceutical composition of the present invention

Ginsenoside Rg₁ with a purity of 85.5% and ginsenoside Rb₁ with a purity of 85.6% prepared in the above preparation example 2 were combined in a weight ratio of 10:1. The resulting mixture was prepared into dripping pills according to conventional preparation methods.

### Preparation Example 7: preparation of pills of the pharmaceutical composition of the present invention

Ginsenoside Rg₁ with a purity of 85.5% prepared in the above preparation example 2 and ginsenoside Rb₁ with a purity of 92.3% prepared in the above preparation example 3 were combined in a weight ratio of 2:1. The resulting mixture was prepared into pills according to conventional preparation methods.

### Preparation Example 8: preparation of syrup of the pharmaceutical composition of the present invention

Ginsenoside Rg₁ with a purity of 91.5% prepared in the above preparation example 3 and ginsenoside Rb₁ with a purity of 99.6% prepared in the above preparation example 1 were combined in a weight ratio of 5:3. The resulting mixture was prepared into syrup according to conventional preparation methods.

### Preparation Example 9: preparation of mixture of the pharmaceutical composition of the present invention

Ginsenoside Rg₁ with a purity of 85.5% and ginsenoside Rb₁ with a purity of 85.6% prepared in the above preparation example 2 were combined in a weight ratio of 1:10. The resulting mixture was prepared into mixture according to conventional preparation methods.

### Preparation Example 10: preparation of granules of the pharmaceutical composition of the present invention

Ginsenoside Rg₁ with a purity of 99.5% and ginsenoside Rb₁ with a purity of 99.6% prepared in the above preparation example 1 were combined in a weight ratio of 10:1. The resulting mixture was prepared into granules according to conventional preparation methods.

### Preparation Example 11: preparation of capsules of the pharmaceutical composition of the present invention

Ginsenoside Rg₁ with a purity of 91.5% prepared in the above preparation example 3 and ginsenoside Rb₁ with a purity of 85.6% prepared in the above preparation example 2 were combined in a weight ratio of 8:1. The resulting mixture was prepared into capsules according to conventional preparation methods.

### Test Example 1: The effect on the nerve and the expression of cell adhesion molecule-1 (ICAM-1) in rats

### 1 Test material:

1.1 drugs: the powder for injection prepared in preparation example 1 of the present invention, 210mg/bottle (containing 120mg of ginsenoside Rg₁ and 90mg of ginsenoside Rb₁); the injection prepared in example 2 of the present invention, 70mg/ml (containing 30mg of ginsenoside Rg₁ and 40mg of ginsenoside Rb₁); the powder for injection prepared in example 3 of the present invention, 210mg/bottle (containing 90mg of ginsenoside Rg₁ and 120mg of ginsenoside Rb₁); Xuesaitong injection, 100mg total saponins of *Panax notoginseng*/bottle, produced by Kunming Pharmaceutical Group Co., Ltd., batch number: 200701064-13; Xuesaitong for injection, 200mg total saponins of *Panax notoginseng*/bottle, produced by Kunming Pharmaceutical Group Co., Ltd., batch number: 200701001-6; 0.9% of sodium chloride injection, produced by Beijing Double-Crane Pharmaceutical Co., Ltd., batch number: 071121610. ICAM-1 monoclonal antibody, produced by Phamingn Corp. in the United States; immunostain S-P Kit, produced by Fuzhou Maixin company; DAB kit, produced by Fuzhou Maixin company; neutral gum, produced by Shanghai Chemical Reagent Company; phosphate buffered saline (PBS), produced by Sigma Corp. in the United States.
1.2 Test animals: SD rats, common-grade, provided by the animal laboratory of Kunming Pharmaceutical Group Co., Ltd. (license number: SYXK (Yunnan 2005-0006)).

### 2 Test methods:

### 2.1 The establishment of the middle cerebral artery occlusion/reperfusion model in SD rats

The diameter of the nylon sutures chosen (produced in Japan) is 0.25mm, and the length thereof is 40mm. The tips of the sutures were fired into rod shape before use. The middle cerebral artery occlusion (MCAO) ischemia model was established according to the line tied method reported by Zea Longa. SD rats were fasted for 12 hours before surgery, anesthetized with 10% chloral hydrate (350mg/kg body weight) via intraperitoneal injection, and fixed in a supine position. The middle neck was incised, and the right common carotid artery (CCA), inner carotid artery (ICA), external carotid artery (ECA) and the branches of ECA were isolated and exposed. The ECA and its branches were ligated. A nylon suture was inserted from the starting of the stump end of the ECA, and the length of the suture was 18+0.5mm. The suture was backdrawn after 2 hours so that the ball end of the suture was back to the stump end of the ECA, and thus the middle cerebral artery was restored blood supply to achieve reperfusion. The incision was firmly ligated, and the full thickness skin was closed. The rats were kept at room temperature and conventionally fed. The criteria for successful models were that the animals developed right Horner syndrome after waking (Homer sign) and left limb paralysis. The operation of the Sham group was same with the above operations, but 15mm depth of the nylon sutures were inserted.

### 2.2 Test groups and treatment

SD rats (220-240g) were randomly divided into the group of 100mg/kg powder for injection prepared in example 1 of the present invention, the group of 100mg/kg injection prepared in example 2 of the present invention, the group of 100mg/kg powder for injection prepared in example 3 of the present invention, the group of 100mg/kg Xuesaitong for injection, the group of 100mg/kg Xuesaitong injection and the control group and the sham group (n=7, a total of 7 groups). The treatment groups and the normal saline control group were divided into 3 groups (1-, 3- and 7-days group) of 5 rats according to the duration of reperfusion after occlusion. Each of the treatment groups was administered 100mg/kg of separate drug immediately after the recovery of reperfusion via the femoral vein. The 3-days-reperfusion group was administered at 24 hours after first administration, once a day, and the 7-days-reperfusion group was administered after 3 days of continuous administration, once every other day. The control group was given equal volume of saline at the above time points, and the sham surgery group was sacrificed by decapitation at 24 hours after surgery.

### 2.3 Preparation of specimens

At different time points of the reperfusion in each group, SD rats were injected intraperitoneally with 10% chloral hydrate (350mg/kg). After deep anesthesia, the left ventricular was perfused with normal saline, thereafter perfused with 4% paraformaldehyde. After being fixed, the brain was stripped out and placed in 4% paraformaldehyde at 4°C for 10 to 12 hours, and then placed in 10% sucrose solution. After the brain being soaked to sink in the bottom, frozen coronal sections were continuously sliced at -20°C. The thickness of the sections was about 25µm, and the sections were kept in 0.01M PBS solution at 4°C until use.

### 2.4 The determination of immunohistochemistry: the SP immunohistochemical staining method of cell adhesion molecule-1 (ICAM-1), according to Kit instructions.

### 3 The indicators of experimental observation:

The nerve function scores of each of the treatment groups and the control group were assessed by Menzies method at 2 hours, 1 day, 3 and 7 days after thrombosis (embolization resulting from middle cerebral artery occlusion). A score of 0 represents no neurological deficit. A score of 1 represents Horner syndrome on the right side. A score of 2 represents failing of extend of the right forepaw. A score of 3 represents rotation to the left. A score of 4 represents circling to the left. A score of 5 represents death. The effects of each treatment group on the nerve function were observed. The effects of each treatment group on ICAM-1 expression were observed.

### 4 Statistical treatment:

ICAM-1 expression was observed under optical microscope (10×10). The positive staining of individual endothelial cell or endothelial cell clusters was deemed as a positive blood marker. One slice was observed for each patient, and 5 non-overlapping fields of vision were randomly selected in the vascular rich area and observed. The quantitative image analysis was preformed, and the number of the positive microvessels per square millimeter in tissue sections was measured. The data were expressed as mean±standard deviation (x±S). Statistical analysis was performed using t test and ANOVA.

### 5. Test results:

**Table 1. The effect on the nerve function scores in rats (x±S)**

| Group | Ischemia for 2 hours | Reperfusion for 1 day | Reperfusion for 3 days | Reperfusion for 7 days |
|---|---|---|---|---|
| Normal saline | 3.36±0.42 | 3.22±0.16 | 3.20±0.18 | 3.02±0.12 |
| Powder for injection according to Example 1 | 2.94±0.32 | 2.82±0.18* | 2.64±0.23* | 2.53±0.22* |
| Injection according to Example 2 | 2.88±0.16 | 2.76±0.26* | 2.58±0.16* | 2.42±0.14* |
| Powder for injection according to Example 3 | 2.92±0.28 | 2.81±0.21* | 2.62±0.32* | 2.51±0.26* |
| Xuesaitong injection | 2.86±0.18 | 2.72±0.24* | 2.58±0.20* | 2.49±0.12* |
| Xuesaitong for injection | 2.91±0.16 | 2.78±0.21* | 2.64±0.25* | 2.52±0.18* |

| | | | | |
|---|---|---|---|---|
| Note: n=5, x±s, *P<0.05, compared with the normal saline group | | | | |

Table 1 suggested the preparations of Examples 1-3 according to the present invention, Xuesaitong injection and Xuesaitong for injection had no significant difference compared with the saline control group after ischemia for 2 hours (P <0.05), then the nerve function of each group was recovered at different degrees. The preparations of Examples 1-3 according to the present invention, Xuesaitong injection and Xuesaitong for injection may improve the brain damage symptoms caused by cerebral ischemia reperfusion, and had significant difference compared with the saline control group (P <0.05).

**Table 2. The effect on the expression of ICAM-1 at different time of cerebral ischemia (x±S)**

| Group | Reperfusion for 1 day | Reperfusion for 3 days | Reperfusion for 7 days |
|---|---|---|---|
| Normal saline | 34.2±5.6 | 39.2±7.8 | 56.4±9.8 |
| Powder for injection according to Example 1 | 13.8±1.8** | 19.9±2.2* | 30.8+4.0* |
| Injection according to Example 2 | 13.4±2.6** | 18.5±1.6* | 29.6±3.2* |
| Powder for injection according to Example 3 | 14.2±2.1** | 19.4±3.2* | 31.1±2.6* |
| Xuesaitong injection | 13.2±2.4** | 18.8±2.0* | 29.4±2.1* |
| Xuesaitong for injection | 13.6±1.9** | 18.2+2.2* | 30.6±2.8* |

| | | | |
|---|---|---|---|
| Note: n=5, x±s, *P<0.05, **P<0.01, compared with the normal saline group | | | |

It was observed that ICAM-1-positive area mainly manifested the microvascular surrounding the infarction area, while no obvious positive reaction was found in the infarction center. No obvious ICAM-1-positive staining vascular was presented in the endothelial cells of bilateral cerebral hemispheres in the sham group and the negative control group. There were ICAM-1-positive vessels at different degrees at different time points of reperfusion in the ischemic area. The numbers of ICAM-1-positive vessels in the groups of the preparations of Examples 1-3 according to the present invention, Xuesaitong injection and Xuesaitong for injection were significantly reduced compared with the normal saline group.

### Test Example 2 The effect on myocardial ischemia

### 1 Test material:

1.1 drugs: the powder for injection prepared in example 1 of the present invention; the injection prepared in example 2 of the present invention; the powder for injection prepared in example 3 of the present invention; Xuesaitong injection, 100mg/bottle, produced by Kunming Pharmaceutical Group Co., Ltd., batch number: 200701064-13; Xuesaitong for injection, 200mg/bottle, produced by Kunming Pharmaceutical Group Co., Ltd., batch number: 200701001-6.
1.2 Test animals: SD rats, common-grade, provided by the animal laboratory of Kunming Pharmaceutical Group Co., Ltd. (license number: SYXK (Yunnan 2005-0006)).

### 2 Test methods:

### 2.1 The establishment of myocardial ischemia model

At 0 minute after intraperitoneal injection of isoproterenol hydrochloride (3ml/kg), stable ST segment depression over 0.2mV may be found, indicating that a typical model of myocardial ischemia was established.

### 2.2 Pharmacodynamic observations:

Drugs were slowly injected via the tail vein at a dose of 100mg/kg. For The nitroglycerin, the dose was 0.17mg/kg. The electrocardiogram was recorded at 5min, 10min, 25min after injection, and the ST segment shift and the T wave changes were observed.

### 2.3 Determination of biochemical indicators:

At 30min after injecting 100mg/kg dose of drugs, the hearts were rapidly taken out. The changes of superoxide dismutase (SOD), catalase(CAT) and lipid peroxide (LPO) were determined and compared. The LPO content was determined using TBA method. The SOD content was determined using riboflavin-NBT method. The CAT level was measured by H₂O₂.

### 2.3 Analysis of experimental data:

Experimental data were presented as mean±standard deviation (x±S), the degree of difference was analyzed using t test.

### 3 Experimental results:

The normal indicators of SD rats were used as controls. The results showed that the ST segment significantly depressed over 0.2mv, i.e., from 0mv to 0.275mv, under the effect of isoproterenol hydrochloride, indicating that the model of myocardial ischemia caused by the isoproterenol hydrochloride was established. In addition, the QT interval was prolonged, and the T wave and the R wave were decreased. The ST segment shift was extremely significant compared with that of the normal group. The QRS interval was reduced from 38.96ms to 29.65ms, and the R wave was reduced from 0.69mv to 0.39mv. These effects were significant compared with that of the normal rats.

**Table 3. ECG indicators of normal rats and rats treated with isoproterenol hydrochloride**

| | HR | R wave | QRS | ST shift | T wave | QT |
|---|---|---|---|---|---|---|
| | (time/min) | (mv) | (ms) | (mv) | (mv) | (ms) |
| Normal indicators | 160.25±18.58 | 0.69±0.21 | 38.96±2.42 | 0 | 0.156±0.03 | 154.55±14.96 |
| Indicators after the effect of isoproterenol hydrochloride | 175.52±13.48 | 0.39±0.11* | 29.65±0.82* | -0.275±0.02** | 0.116±0.05 | 174.54±12.69 |

| | | | | | | |
|---|---|---|---|---|---|---|
| Note: N=5, *P<0.05, **P<0.01, compared with the normal indicators | | | | | | |

**Table 4. ECG indicators of rats at 5, 10 and 25mins after being treated with various drugs**

| Time | Group | HR | R wave | QRS | ST shift | T wave | QT |
|---|---|---|---|---|---|---|---|
| | | (time/min) | (mv) | (ms) | (mv) | (mv) | (ms) |
| 5min | nitroglycerin | 192.25±8.58 | 0.68±1.21 | 34.61±0.42 | -0.04±1.47 | 0.146±0.75 | 114.58±0.96 |
| | Powder for injection according to Example 1 | 190.36±3.52* | 0.60±0.25 | 31.76±0.32 | -0.09±0.02* | 0.15±0.15 | 104.24±0.36** |
| | Injection according to Example 2 | 188.24±4.16* | 0.580±0.31 | 29.88±0.28 | -0.08±0.01* | 0.145±0.19 | 102.33±0.29** |
| | Powder for injection according to Example 3 | 185.22±4.16* | 0.62±0.34 | 28.46±0.41 | -0.08±0.03* | 0.140±0.21 | 99.84+0.43** |
| | Xuesaitong injection | 183.98±4.65* | 0.55±0.21 | 29.86±0.43 | -0.08±0.02* | 0.142±0.18 | 100.28±0.43** |
| | Xuesaitong for injection | 187.38±4.34* | 0.59±0.33 | 30.88±0.46 | -0.09±0.03* | 0.148±0.18 | 102.26±0.29** |
| 10min | nitroglycerin | 180.53±3.56 | 0.76±0.22 | 38.11±1.42 | -0.06±0.02 | 0.124±0.02 | 118.98±0.32 |
| | Powder for injection according to Example 1 | 191.94±3.62 | 0.40±0.28 | 25.16±0.62 | -0.009±0.1** | 0.052±0.35 | 110.94±0.48 |
| | Injection according to Example 2 | 190.12±3.18 | 0.380±0.26 | 24.92±0.58 | -0.008±0.1** | 0.046±0.24 | 109.44±0.32 |
| | Powder for injection according to Example 3 | 188.02±3.16 | 0.42±0.28 | 24.88±0.68 | -0.008±0.1** | 0.040±0.12 | 105.66±0.55 |
| | Xuesaitong injection | 185.16±5.64 | 0.45±0.22 | 25.04±0.48 | -0.008±0.1** | 0.042±0.12 | 104.76±0.38 |
| | Xuesaitong for injection | 189.16±3.36 | 0.39±0.12 | 24.98±0.52 | -0.009±0.1** | 0.044±0.14 | 105.24±0.35 |
| 25min | nitroglycerin | 187.15±6.52 | 0.73±0.62 | 40.221±0.44 | -0.195±0.74 | 0.153±0.052 | 160.92±0.06 |
| | Powder for injection according to Example 1 | 197.38±3.82 | 0.348±0.07 | 25.86±0.06 | 0** | 0.225±0.025 | 134.25±0.08 |
| | Injection according to Example 2 | 198.64±5.18 | 0.352±0.08 | 49.82±0.05 | 0** | 0.245±0.016 | 132.36±0.09 |
| | Powder for injection according to Example 3 | 195.82±4.26 | 0.362±0.09 | 48.42±0.06 | 0** | 0.242±0.022 | 130.96±0.12 |
| | Xuesaitong injection | 193.64±3.64 | 0.415±0.12 | 49.56±0.07 | -0.24±0.004* | 0.234±0.016 | 130.46±0.08 |
| | Xuesaitong for injection | 197.32±4.86 | 0.392+0.08 | 50.04±0.06 | -0.29±0.003* | 0.248±0.018 | 132.86+0.10 |

| | | | | | | | |
|---|---|---|---|---|---|---|---|
| **Note: N=5, *P<0.05, **P<0.01, compared with the nitroglycerin group** | | | | | | | |

The preparations prepared in Examples 1-3, Xuesaitong injection and Xuesaitong for injection can significantly antagonize the ST segment depression induced by isoproterenol (>0.2mv) in myocardial ischemia model, and these effects were significantly higher than that of nitroglycerin. The preparations prepared in Examples 1-3, Xuesaitong injection and Xuesaitong for injection improved the flat or low-lying of T wave (<0.12mv), the R wave reduction and the prolonged QT interval to some extent.

**Table 5. The effect of various drugs on the biochemical indicators**

| Indicators | Normal | isoproterenol hydrochloride | Powder for injection according to Example 1 | Injection according to Example 2 | Powder for injection according to Example 3 | Xuesaitong injection | Xuesaitong for injection |
|---|---|---|---|---|---|---|---|
| SOD | 19.87±5.82 | 21.68±6.20 | 31.30±1.28* | 32.12±1.46* | 30.96±1.28* | 29.62±1.96* | 30.02±1.68* |
| CAT (10⁻⁴kg/Hb) LPO | 3.75±2.80 | 2.94±2.02 | 6.42±0.05* | 6.38±0.06* | 6.44±0.07* | 6.32±8.16* | 5.98±7.25* |
| | 0.315±1.26 | 0.245±0.05 | 0.242±0.04* | 0.236±0.03* | 0.238±0.04* | 0.244±0.04* | 0.246±0.05* |

| | | | | | | | |
|---|---|---|---|---|---|---|---|
| Note: N=5, *P<0.05, **P<0.01, compared with the normal group | | | | | | | |

It could be seen from Table 5, the preparations prepared in Examples 1-3, Xuesaitong injection and Xuesaitong for injection could increase the level of superoxide dismutase ((SOD) and catalase (CAT), and thus had protective effects on the myocardial cells.

### Test Example 3. the preventive effect on atherosclerosis (AS) in rabbits

### 1 Test materials

### 1.1 Reagents and Drugs:

The preparations prepared in Examples 1-3, Xuesaitong injection and Xuesaitong for injection; total cholesterol (TC) kit, triglyceride (TG) kit, low density lipoprotein cholesterol (LDL-C) kit (Beijing Chemical Reagent Factory clinical Reagent Factory Accessories); superoxide dismutase (SOD) kit, malondialdehyde (MDA) kit (Nanjing Jiancheng Bioengineering Institute.)

The formula for high fat fodder: basal fodder 84.5%, eggs 10%, lard 5%, cholesterol 0.5%.

1.2 Animals: Japanese white rabbits, weighing 1.8-2.0kg, common-grade, provided by the animal laboratory of Kunming Pharmaceutical Group Co., Ltd.(license number: SYXK (Yunnan 2005-0006)).

### 2 Test method:

### 2.1 The establishment of hyperlipemia model, grouping and administration

84 Japanese white rabbits were fasted for 12h. Blood was collected via the ear vein, and the serum TG, TC, LDL-C levels were determined. The rabbits were divided at random into 14 groups according to the TC levels, 6 rabbits per group. The rabbits in the blank control group were fed with basal fodder, 120-150g per rabbit. The rabbits in the AS model group were fed with high fat fodder, 120-150g per rabbit. The rabbits in the low dose groups of the preparations prepared in Examples 1-3, Xuesaitong injection and Xuesaitong for injection were fed with high fat fodder and orally administered 60mg/kg/d of the preparations prepared in Examples 1-3, Xuesaitong injection and Xuesaitong for injection. The rabbits in the high dose groups of the preparations prepared in Examples 1-3, Xuesaitong injection and Xuesaitong for injection were fed with high fat fodder and orally administered 120mg/kg/d of the preparations prepared in Examples 1-3, Xuesaitong injection and Xuesaitong for injection. The experiment was carried out for 12 weeks.

### 2.2 Detection of the indicators:

The rabbits were fasted for 12 hours before administration and at the end of the 12th week. 3 ml of blood was collected via the ear vein, and the serum was separated. The serum TG, TC, LDL-C and MDA level and the SOD activity were determined according to the instructions in kits. The animals were killed after the blood was collected. The aorta was taken out and the artery was cut open in longitude, fixed in 10% formaldehyde solution, stained with Sudan IV. After taking pictures using a digital camera, the pictures were analyzed using Image Pro Plus software and the area ratio of AS plaque to the entire artery was calculated. The extent of the aorta atherosclerotic plaque was graded with reference to "pharmacological experiments methodology" edited by Xu Shuyun: grade I (no lesion); grade II (the fraction of lesion is 1-25%); grade III (the fraction of lesion is 26-50%); grade IV (the fraction of lesion is 51-75%); grade V (the fraction of lesion is 76-100%).

### 2.3 Statistical analysis:

The data were presented as x±s. Analysis of variance for data of each group was performed using SAS software.

### 3 Experimental Results:

### 3.1 The effect of each treatment group on blood lipid in rabbits

All components of blood lipid in each group had no significant difference before the experiment. After 12 weeks, the TG, TC and LDL-C levels in the AS model group were significantly higher than those of the control group (P<0.01). For the preparations prepared in Examples 1-3, Xuesaitong injection and Xuesaitong for injection, the TG, TC and LDL-C levels in the high dose group (P<0.01) and the low dose group had no statistical difference with those of the model group.

**Table 6. The effects on the TG, TC and LDL-C levels in rabbits (x±s, n=5)**

| Group | TC(mmol/L) | | TG(mmol/L) | | LDL-C(mmol/L) | |
|---|---|---|---|---|---|---|
| | 0 week | 12 week | 0 week | 12 week | 0 week | 12 week |
| Blank control group | 3.50±1.20 | 3.98±1.35 | 0.62±0.15 | 0.86±0.24 | 0.72±0.17 | 0.68±0.18 |
| AS model group | 3.42±0.54 | 24.88±4.92* | 0.59±0.14 | 4.76±0.12* | 0.84±0.22 | 15.36±2.12* |
| Powder for injection according to Example 1 (low dose) | 3.42±0.56 | 19.86±4.28 | 0.58±0.15 | 3.42±0.84 | 0.83±0.28 | 12.72±2.76 |
| Injection according to Example 2 (low dose) | 3.48±0.52 | 20.06+4.64 | 0.56±0.12 | 3.36±0.72 | 0.74±0.21 | 13.12±3.26 |
| Powder for injection according to Example 3 (low dose) | 3.34±0.46 | 18.96±4.46 | 0.60±0.14 | 3.55±0.78 | 0.78+0.30 | 12.98±2.84 |
| Xuesaitong injection (low dose) | 3.40±0.46 | 18.76±3.68 | 0.52±0.18 | 3.54±0.68 | 0.79±0.26 | 12.85±3.12 |
| Xuesaitong for injection (low dose) | 3.44±0.58 | 19.18±3.96 | 0.54±0.12 | 3.64±0.82 | 0.85±0.32 | 13.22±2.84 |
| Powder for injection according to Example 1 (high dose) | 3.28±0.66 | 9.35±2.16* | 0.68±0.16 | 2.32±0.84* | 0.96±0.34 | 4.68±1.42* |
| Injection according to Example 2 (high dose) | 3.22±0.48 | 9.72±3.26* | 0.46±0.12 | 2.18±0.76* | 0.85±0.24 | 4.52±1.38* |
| Powder for injection according to Example 3 (high dose) | 3.28±0.42 | 9.16±3.86* | 0.58±0.18 | 2.44±0.78* | 0.82±0.22 | 4.74±2.76* |
| Xuesaitong injection (high dose) | 3.36±0.52 | 9.84±4.54* | 0.48±0.16 | 2.64±0.92* | 0.76±0.24 | 4.86±2.46* |
| Xuesaitong for injection (high dose) | 3.24±0.48 | 9.92±4.76* | 0.56±0.12 | 2.34±0.58* | 0.80±0.24 | 4.36±2.43* |

| | | | | | | |
|---|---|---|---|---|---|---|
| Note: *P<0.01, compared with the blank control group; *P<0.01, compared with the AS model group | | | | | | |

### 3.2 The effect of each treatment group on serum SOD activity and MDA levels in rabbits

The serum MDA level of rabbits in the AS model group was significantly higher than that of the control group, and the SOD activity was significantly decreased. For the powder for injection of example 1, the injection of example 2, the powder for injection of Example 3, Xuesaitong injection and Xuesaitong for injection, the levels of serum MDA at high dose were significantly lower than that of the AS model group (P<0.01), while SOD activity was significantly higher than that of the AS model group (P<0.01). For the powder for injection of example 1, the injection of example 2, the powder for injection of example 3, Xuesaitong injection and Xuesaitong for injection, the levels of serum MDA at low dose were significantly lower than that of the AS model group (P<0.05), while SOD activity had no difference with that of the AS model group.

**Table 7. The effect on serum SOD activity and MDA levels in rabbits (x±s, n=5)**

| Group | MDA (umol/L) | SOD/U/L |
|---|---|---|
| blank control group | 6.88±1.24 | 372.24±18.76 |
| AS model group | 25.42±4.72* | 204.32±15.46* |
| powder for injection according to Example 1 (low dose) | 20.34+3.46 | 218.86±16.12 |
| injection according to Example 2 (low dose) | 19.86±2.72 | 214.58±15.84 |
| powder for injection according to Example 3 (low dose) | 21.04±3.64 | 221.18±15.54 |
| Xuesaitong injection (low dose) | 20.24±3.16 | 212.44±15.16 |
| Xuesaitong for injection (low dose) | 21.16±2.88 | 224.16±17.02 |
| powder for injection according to Example 1 (high dose) | 14.44±3.42* | 314.82±9.88* |
| injection according to Example 2 (high dose) | 15.51±3.16* | 318.12±10.12* |
| powder for injection according to Example 3 (high dose) | 14.46±3.65* | 320.14±10.24* |
| Xuesaitong injection (high dose) | 16.24±4.12* | 306.25±9.18* |
| Xuesaitong for injection (high dose) | 25.84±3.82* | 311.15±10.36* |

| | | |
|---|---|---|
| Note: *P<0.01, compared with the blank control group; *P<0.01, compared with the AS model group | | |

### 3.3 Pathological examination

The aortic intimas of the rabbits in the blank group were smooth and flat, and no atheromatous plaque was present. There was a large number of protruding plaques on the aortic intimas of rabbits in the AS model group, some of which became confluent, and the endothelial cells in the plaques were significantly proliferated, in addition, a large number of foam cells aggregated, and the intimas became thickened significantly, some cells were completely occupied by confluent lipid droplets. Small vacuoles were presented in the cytoplasm, and the vacuoles were surrounded by a clear proliferation of collagen fibers, the grade of AS plaque was significantly higher than that of other groups. The aortic endothelial cells was still intact in PNS high dose group, and a small amount of foam cells were seen under intimas, proliferation of smooth muscle cells was not found, and the grade of aortic lesion was significantly reduced.

The above tests demonstrated that the preparations prepared in Example 1, Example 2, and Example 3 and Xuesaitong injection, Xuesaitong for injection could prevent atherosclerosis by regulating lipid metabolism and anti-oxygen free radicals and other means.

### Test Example 4. Comparison of the toxicity of the drugs according to the present invention with Panax notoginseng saponins injection

A comparison of the toxicity of the drugs according to the present invention with *Panax notoginseng* saponins injection was performed with reference to the abnormal toxicity test and the acute toxicity test in "Chinese Pharmacopoeia" (2005 edition).

**Table 8. The results of the acute toxicity (LD₅₀)**

| Drugs tested | The powder for injection of example 1 | The injection of example 2 | The powder for injection of example 3 | Xuesaitong injection | Xuesaitong for injection |
|---|---|---|---|---|---|
| LD₅₀ | 482mg/kg | 428mg/kg | 465mg/kg | 293mg/kg | 315mg/kg |

**Table 9. The results of the abnormal toxicity**

| Drugs tested | The powder for injection of example 1 | The injection of example 2 | The powder for injection of example 3 | Xuesaitong injection | Xuesaitong for injection |
|---|---|---|---|---|---|
| Concentration | 25mg/ml | 23mg/ml | 25mg/ml | 11mg/ml | 12mg/ml |

By comparing the results of above two tests, it was found that the products of the present invention brought about unexpected improvement both in toxicity and abnormal toxicity, and thus the safety of the products according to the present invention was enhanced markedly. Therefore, the purpose of the present invention can be achieved.

### Test Example 5. Comparison of the hemolysis of the drugs according to the present invention with Panax notoginseng saponins injection

The hemolysis test was performed according to the test methods of "technical guidelines for irritation and hemolysis study on traditional Chinese medicine and natural medicine", in order to observe the hemolysis and coagulation of the drugs according to the present invention and related *Panax notoginseng* saponins injection at different concentrations.

### 1 Test Materials:

1.1 Drugs: the powder for injection prepared in example 1 of the present invention; the injection prepared in example 2 of the present invention; the powder for injection prepared in example 3 of the present invention; Xuesaitong injection, 100mg/bottle, produced by Kunming Pharmaceutical Group Co., Ltd., batch number: 200701064-13; Xuesaitong for injection, 200mg/bottle, produced by Kunming Pharmaceutical Group Co., Ltd., batch number: 200701001-6.

Preparation of 2% red blood cells suspension: 20-30ml of blood was drawn from a rabbit. After fibrinogen being removed, the blood was washed with about 10 times volume of sodium chloride injection, then centrifuged at 1500r/min for 15min. The supernatant was discarded, and the centrifugation step was repeated 2-3 times until the supernatant was not red. The supernatant was discarded, and the resulting red blood cells were prepared into 2% suspension with sodium chloride injection solution for the use of experiments.

Preparation of the drugs to be tested: the powder for injection prepared in example 1 of the present invention, the powder for injection prepared in example 3 of the present invention and Xuesaitong for injection were weighed precisely. Sodium chloride injection solution was added thereto to obtain solutions containing 15mg and 25mg drug per 1ml solution respectively. A suitable amount of the injection prepared in example 2 of the present invention and Xuesaitong injection were taken. Sodium chloride injection solution was added thereto to obtain solutions containing 15mg and 25mg drug per ml solution respectively.

### 2 Test Method:

7 test tubes were taken respectively, wherein tubes No. 1-5 contained drugs to be tested, tube No. 6 was the negative control tube (only containing 2% red blood cell suspension and sodium chloride injection solution), tube No. 7 was the positive control tube (only containing 2% red blood cell suspension and distilled water). 2% red blood cell suspension, sodium chloride injection solution and distilled water were added thereto according to the following table. The tubes were placed in a thermostatic water bath and incubated at 37°C±0.5°C immediately after mixing well. At first, the tubes were observed every 15 minutes, and observed at 1-hour interval after 1h. The tubes were observed continuously for 24 hours.

**Table 10. The observation of hemolysis and coagulation of the inventive drugs and Panax notoginseng saponins injection solution sample**

| test tube No. | 1 | 2 | 3 | 4 | 5 | 6 | 7 |
|---|---|---|---|---|---|---|---|
| 2% red blood cell suspension | 2.5 | 2.5 | 2.5 | 2.5 | 2.5 | 2.5 | 2.5 |
| sodium chloride injection | 2.0 | 2.1 | 2.2 | 2.3 | 2.4 | 2.5 | 0 |
| distilled water | 0 | 0 | 0 | 0 | 0 | 0 | 2.5 |
| drugs tested | 0.5 | 0.4 | 0.3 | 0.2 | 0.1 | 0 | 0 |

If the solution in the test tube was red and clear, and there was no or a small amount of residual red blood cells on the bottom of the tube, it suggested that hemolysis occurred. If all of the red blood cells sinked down and the supernatant was colorless and clear, it suggested that no hemolysis occurred.

If red brown flocculent precipitation appeared in the solution and did not disappeared after shaking, it suggested that red blood cells coagulation occured. If red blood cells coagulation occured, it could be further determined whether the coagulation was true according to the following method. If the coagulation could evenly disperse after shaking the test tube, or if the coagulated red blood cells can be broken up by observing under a microscope when the coagulation was placed on a slide and 2 drops of sodium chloride injection solution was dropped on the edge of the slide, the coagulation should be considered as false coagulation, if not, the coagulation should be considered as true coagulation.

### 3. Determination of the results

Where no hemolysis and coagulation occurred in the negative control tube and hemolysis occurred in the positive control tube, if no hemolysis and coagulation occurred in the solution containing drugs tested of the third tube (0.3ml) within 3 hours, the drugs tested were considered to meet requirements; if hemolysis and coagulation occurred in the solution containing drugs tested of the third tube (0.3ml) within 3 hours, the drugs tested were considered not to meet requirements.

### 4 Results:

For the preparations of Example 1, Example 2 and Example 3 according to the present invention, at the concentration up to 25mg/ml, no hemolysis and coagulation occurred in the solution of the third tube (0.3ml) within 3 hours. Hemolysis began in the first tube (0.5ml) at 3 hours and in the second tube (0.4ml) at 5 hours. At the concentration of 15mg/ml, no hemolysis occurred. For Xuesaitong injection and Xuesaitong for injection, at the concentration up to 15mg/ml, hemolysis had occurred in the third tube (0.3ml) at 3 hours.

In terms of the indicator of hemolysis, the hemolytic issues of traditional Chinese medicine injection solutions are also extremely important, especially, the hemolysis of saponins has been the main reason why a lot of saponins could not be prepared into injections. The hemolysis of the present products is obviously reduced compared with *Panax notoginseng* saponins injection, so the safety is greatly enhanced.

## Claims

1. A pharmaceutical composition for use in the treatment or prevention of cardio-cerebro vascular diseases, **characterized in that** the pharmaceutical composition consists of a ginsenoside Rg₁ and a ginsenoside Rb₁ as active ingredients and a pharmaceutically acceptable carrier, wherein the weight ratio of ginsenoside Rg₁ to ginsenoside Rb₁ is 1:10 to 10:1, and the total amount of ginsenoside Rg₁ and ginsenoside Rb₁ is 85% to 99.99%, based on the total weight of the pharmaceutical composition.

2. The pharmaceutical composition for use in the treatment or prevention of cardio-cerebro vascular diseases according to claim 1, **characterized in that** the weight ratio of the ginsenoside Rg₁ to the ginsenoside Rb₁ is 3:5 to 5:1.

3. The pharmaceutical composition for use in the treatment or prevention of cardio-cerebro vascular diseases according to claim 1, **characterized in that** the weight ratio of the ginsenoside Rg₁ to the ginsenoside Rb₁ is 1:1.5 to 1.5:1.

4. The pharmaceutical composition for use in the treatment or prevention of cardio-cerebro vascular diseases according to claim 1, **characterized in that** the weight ratio of the ginsenoside Rg₁ to the ginsenoside Rb₁ is about 1:1.

5. The pharmaceutical composition for use in the treatment or prevention of cardio-cerebro vascular diseases according to any one of claims 1-4, **characterized in that** the pharmaceutically acceptable carrier is selected from excipients, fillers, binders, disintegrating agents, lubricants, antioxidants, coating agents, coloring agents, fragrances, surfactants and any combination thereof.

6. The pharmaceutical composition for use in the treatment or prevention of cardio-cerebro vascular diseases according to any one of claims 1-5, **characterized in that** the pharmaceutical composition is formulated into injection, powder for injection, enteric-coated tablets, pills, powder, granules, mixtures, syrup, capsules or dripping pills.

7. The pharmaceutical composition for use in the treatment or prevention of cardio-cerebro vascular diseases according to any one of claims 1-6, **characterized in that** the cardio-cerebro vascular disease is selected from obstruction of collaterals by blood stasis, apoplexy and hemiplegia, chest distress and cardiodynia, obstruction of central retinal vein.

8. A method for preparing the pharmaceutical composition according to any one of claims 1-7, **characterized in that** the method comprises the step of combining ginsenoside Rg₁ with ginsenoside Rb₁ in a weight ratio of 1:10 to 10:1, wherein the purity of ginsenoside Rg₁ used is 85% to 99.99%, and the purity of ginsenoside Rb₁ used is 85% to 99.99%.

9. A kit for use in the improvement, treatment or prevention of cardio-cerebro vascular diseases, **characterized in that** the kit comprises the pharmaceutical composition according to any one of claims 1-7.

10. The kit for use in the improvement, treatment or prevention of cardio-cerebro vascular diseases according to claim 9, wherein the kit further comprises a drug delivery device and/or instructions for administration.

## Patentansprüche

1. Pharmazeutische Zusammensetzung zur Anwendung in der Behandlung oder Vorbeugung von kardio-zerebrovaskulären Erkrankungen, **dadurch gekennzeichnet, dass** die pharmazeutische Zusammensetzung aus einem Ginsenosid Rg₁ und einem Ginsenosid Rb₁ als aktive Bestandteile und einem pharmazeutisch akzeptablen Träger besteht, wobei das Gewichtsverhältnis von Ginsenosid Rg₁ zu Ginsenosid Rb₁ 1:10 bis 10:1 beträgt, und wobei die Gesamtmenge von Ginsenosid Rg₁ und Ginsenosid Rb₁ 85% bis 99.99% ist, bezogen auf das Gesamtgewicht der pharmazeutischen Zusammensetzung.

2. Pharmazeutische Zusammensetzung zur Anwendung in der Behandlung oder Vorbeugung von kardio-zerebrovaskulären Erkrankungen gemäss Anspruch 1, **dadurch gekennzeichnet, dass** das Gewichtsverhältnis des Ginsenosids Rg₁ zum Ginsenosid Rb₁ 3:5 bis 5:1 beträgt.

3. Pharmazeutische Zusammensetzung zur Anwendung in der Behandlung oder Vorbeugung von kardio-zerebrovaskulären Erkrankungen gemäss Anspruch 1, **dadurch gekennzeichnet, dass** das Gewichtsverhältnis des Ginsenosids Rg₁ zum Ginsenosid Rb₁ 1:1.5 bis 1.5:1 beträgt.

4. Pharmazeutische Zusammensetzung zur Anwendung in der Behandlung oder Vorbeugung von kardio-zerebrovaskulären Erkrankungen gemäss Anspruch 1, **dadurch gekennzeichnet, dass** das Gewichtsverhältnis des Ginsenosids Rg₁ zum Ginsenosid Rb₁ ungefähr 1:1 beträgt.

5. Pharmazeutische Zusammensetzung zur Anwendung in der Behandlung oder Vorbeugung von kardio-zerebrovaskulären Erkrankungen gemäss einem der Ansprüche 1 bis 4, **dadurch gekennzeichnet, dass** der pharmazeutisch akzeptable Träger ausgewählt ist aus Hilfsstoffen, Füllmitteln, Bindemitteln, Sprengmitteln, Gleitmitteln, Antioxidantien, Überzugsmitteln, Farbstoffen, Duftstoffen, Tensiden und einer beliebigen Kombination hiervon.

6. Pharmazeutische Zusammensetzung zur Anwendung in der Behandlung oder Vorbeugung von kardio-zerebrovaskulären Erkrankungen gemäss einem der Ansprüche 1 bis 5, **dadurch gekennzeichnet, dass** die pharmazeutische Zusammensetzung formuliert ist als Injektion, Pulver für eine Injektion, magensaftresistente Tabletten, Pillen, Pulver, Granulate, Mischungen, Sirup, Kapseln oder Tropfpillen.

7. Pharmazeutische Zusammensetzung zur Anwendung in der Behandlung oder Vorbeugung von kardio-zerebrovaskulären Erkrankungen gemäss einem der Ansprüche 1 bis 6, **dadurch gekennzeichnet, dass** die kardio-zerebrovaskuläre Erkrankung ausgewählt ist aus Obstruktionen von Kollateralen durch Blutstase, Apoplexie und Hemiplegie, Brustbeschwerden und Kardiodynie, Obstruktion der zentralen Netzhautvene.

8. Verfahren zur Herstellung der pharmazeutischen Zusammensetzung nach einem der Ansprüche 1-7, **dadurch gekennzeichnet, dass** das Verfahren den Schritt des Kombinierens von Ginsenosid Rg₁ mit Ginsenosid Rb₁ in einem Gewichtsverhältnis von 1:10 bis 10:1 umfasst, wobei die Reinheit des verwendeten Ginsenosids Rg₁ 85% bis 99.99% beträgt und die Reinheit des verwendeten Ginsenosids Rb₁ 85% bis 99.99% beträgt.

9. Kit zur Anwendung in der Verbesserung, Behandlung oder Vorbeugung von kardio-zerebrovaskulären Krankheiten, **dadurch gekennzeichnet, dass** das Kit die pharmazeutische Zusammensetzung nach einem der Ansprüche 1 bis 7 umfasst.

10. Kit zur Anwendung in der Verbesserung, Behandlung oder Vorbeugung von kardio-zerebrovaskulären Krankheiten nach Anspruch 9, wobei das Kit ausserdem eine Medikamentenabgabevorrichtung und/oder Instruktionen für die Verabreichung umfasst.

## Revendications

1. Une composition pharmaceutique pour utilisation dans le traitement ou la prévention de maladies cardio-cérébrovasculaires, **caractérisée en ce que** la composition pharmaceutique consiste d'un ginsenoside Rg1 et d'un ginsenoside Rb1 en tant qu'ingrédient actif et un véhicule pharmaceutiquement acceptable, dans laquelle le rapport en poids entre le ginsenoside Rg1 et le ginsenoside Rb1 est de 1:10 à 10:1, et la quantité totale de ginsenoside Rg1 et de ginsenoside Rb1 est 85% à 99.99% à base du poids total de la composition pharamaceutique.

2. La composition pharmaceutique pour utilisation dans le traitement ou la prévention de maladies cardio-cérébrovasculaires selon la revendication 1, **caracterisée en ce que** le rapport en poids entre le ginsénoside Rg1 et le ginsénoside Rb1 est de 3:5 à 5:1.

3. La composition pharmaceutique pour utilisation dans le traitement ou la prévention de maladies cardio-cérébrovasculaires selon la revendication 1, **caracterisée en ce que** le rapport en poids entre le ginsénoside Rg1 et le ginsénoside Rb1 est de 1:1.5 à 1.5:1.

4. La composition pharmaceutique pour utilisation dans le traitement ou la prévention de maladies cardio-cérébrovasculaires selon la revendication 1, **caracterisée en ce que** le rapport en poids entre le ginsénoside Rg1 et le ginsénoside Rb1 est d'environ 1:1.

5. La composition pharmaceutique pour utilisation dans le traitement ou la prévention de maladies cardio-cérébrovasculaires selon l'une quelconque des revendications 1 à 4, **caractérisée en ce que** le véhicule pharmaceutiquement acceptable est choisi parmi d'excipients, de charges, de liants, d'agents de désintégration, de lubrifiants, d'antioxydants, d'agents de revêtement, d'agent colorants, de parfums, d'agents tensioactifs ou quelconque combinaison de ceux-ci.

6. La composition pharmaceutique pour utilisation dans le traitement ou la prévention de maladies cardio-cérébrovasculaires selon l'une quelconque des revendications 1 à 5, **caractérisée en ce que** la composition pharmaceutique est formulée pour une injection, une poudre pour l'injection, comprimé enrobé entérique, pilules, poudre, granulés, mélanges, sirop, capsules ou pilules goutte.

7. La composition pharmaceutique pour utilisation dans le traitement ou la prévention de maladies cardio-cérébrovasculaires selon l'une quelconque des revendications 1 à 6, **caractérisée en ce que** la maladie cardio-cérébrovasculaire est choisie parmi obstruction de collatérale par stase sanguine, apoplexie et hémiplégie, détresse thoracique et cardiodynie, obstruction de la rétinienne centrale.

8. Un procédé pour la préparation d'une composition pharmaceutique selon l'une quelconque des revendications 1 à 7, **caractérisée en ce que** le procédé comprend l'étape de combiner le ginsénoside Rg1 avec le ginsénoside Rb1 dans un rapport en poids de 1:10 à 10:1, dans lequel la pureté du ginsénoside Rg1 utilisé est de 85% à 99.99% et la pureté du ginsénoside Rb1 utilisé est de 85% à 99.99%.

9. Un kit pour l'utilisation dans l'amélioration, le traitement ou la prévention de maladies cardio-cérébrovasculaires, **caractérisé en ce que** le kit comprend la composition pharmaceutique selon l'une quelconque des revendications 1 à 7.

10. Le kit pour l'utilisation dans l'amélioration, le traitement ou la prévention de maladies cardio-cérébrovasculaires selon la revendication 9, dans lequel le kit comprend en outre un dispositif d'administration de médicament et / ou des instructions pour l'administration.
